(19) Europäisches Patentamt
European Patent Office
Office européen des brevets

(11) **EP 2 111 423 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**04.01.2017 Patentblatt 2017/01**

(45) Hinweis auf die Patenterteilung:
**28.07.2010 Patentblatt 2010/30**

(21) Anmeldenummer: **08701336.3**

(22) Anmeldetag: **09.01.2008**

(51) Int Cl.:
*C08G 18/79* (2006.01)     *C08G 18/76* (2006.01)

(86) Internationale Anmeldenummer:
**PCT/EP2008/050171**

(87) Internationale Veröffentlichungsnummer:
**WO 2008/084054 (17.07.2008 Gazette 2008/29)**

(54) **POLYURETHAN-HARTSCHAUMSTOFFE**

POLYURETHANE RIGID FOAMS

MOUSSE DURE DE POLYURÉTHANE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **12.01.2007 EP 07100482**

(43) Veröffentlichungstag der Anmeldung:
**28.10.2009 Patentblatt 2009/44**

(73) Patentinhaber: **BASF SE**
**67056 Ludwigshafen am Rhein (DE)**

(72) Erfinder:
• **SEIFERT, Holger**
**49163 Bohmte (DE)**
• **KLASSEN, Johann**
**32351 Stemwede-Oppendorf (DE)**
• **DU RIEU, Louis**
**NL-6415 KW Bergen op Zoom (NL)**
• **WIEGMANN, Werner**
**32369 Rahden-Wehe (DE)**
• **MATZKE, Günter**
**49356 Diepholz (DE)**

(74) Vertreter: **BASF IP Association**
**BASF SE**
**ZRX-C6**
**67056 Ludwigshafen (DE)**

(56) Entgegenhaltungen:
**EP-A- 0 294 110     EP-A- 1 518 874**

EP 2 111 423 B2

**Beschreibung**

Gegenstand der Erfindung sind Polyurethan-Hartschaumstoffe und ihre Herstellung.

Polyurethan-Hartschaumstoffe sind seit langem bekannt und vielfach beschrieben. Der Einsatz der Polyurethan- Hartschaumstoffe erfolgt vorwiegend zur Wärmeisolation, beispielsweise in Kältegeräten, Transportmitteln oder Gebäuden sowie zur Herstellung von Bauelementen, insbesondere Sandwich-Elementen.

Ihre Herstellung erfolgt üblicherweise durch Umsetzung von Polyisocyanaten, wobei hier zumeist Diphenylmethandiisocyanat (MDI) und insbesondere Mischungen aus Diphenylmethandiisocyanat und den höheren Homologen Polyphenylenpolymethylenpolyisocyanaten (Roh-MDI) eingesetzt werden, mit Verbindungen mit mindestens zwei mit Isocyanatgruppen reaktiven Wasserstoffatomen.

Eine wesentliche Anforderung an die Polyurethan- Hartschaumstoffe ist die Dimensionsstabilität. Dimensionsstabilität bedeutet, dass der Schaum nach der Aushärtung sein Volumen nicht ändert, insbesondere nicht schrumpft. Bei Hartschaumstoffen kann es durch den Schrumpf Hohlräume im Schaum sowie Ablösungen von den Deckschichten geben. Dabei besteht das Schrumpfproblem insbesondere bei großen Formteilen, zu dessen Lösung es keinerlei Hinweise gibt.

Weiterhin werden am Markt zunehmend Schäume verlangt, die eine rasche Entformbarkeit aufweisen. Eine zu lange Entformzeit ist insbesondere für Anwendungen, bei denen der Schaum in relativ dicken Schichten, insbesondere mindestens 60 mm, eingesetzt wird, besonders störend.

Aufgabe der Erfindung war es daher, Polyurethan-Hartschaumstoffe zur Verfügung zu stellen, die gute Verarbeitungseigenschaften, insbesondere eine geringe Entformzeit, und gute Gebrauchseigenschaften, insbesondere eine gute Dimensionsstabilität, aufweisen. Dabei sollten diese vorteilhaften Eigenschaften auch bei niedriger Dichte, vorzugsweise einer Kernrohdichte < 30 g/l, auftreten. Weiterhin sollte den Forderungen des Marktes nach hellen Schaumstoffen entsprochen werden.

In EP 0 294 110 werden Polymethylenpolyphenylenpolyisocyanatgemische offenbart die 0-20% 2-Kern, 40-80% 3-Kern, 5-25% 4- und 5-Kern und 0-20% höhere mehr-Kern MDI enthalten. Diese Polyisocyanate werden zur Herstellung von Polyurethanhartschäumen verwendet.

Die Aufgabe konnte überraschenderweise gelöst werden durch die Verwendung einer Mischung aus Diphenylmethandiisocyanaten und Polyphenylenpolymethylenpolyisocyanaten mit einer speziellen Zusammensetzung als Isocyanatkomponente bei der Herstellung der Schaumstoffe.

Gegenstand der Erfindung sind demzufolge Polyurethan-Hartschaumstoffe gemäß Anspruch 1.

Gegenstand der Erfindung ist weiterhin ein Verfahren zur Herstellung der erfindungsgemäßen Polyurethan-Hartschaumstoffe gemäß Anspruch 12.

Erfindungsgemäß die zur Herstellung der Polyurethan-Hartschaumstoffe eingesetzte Isocyanatkomponente a) weniger als 12 Gew.-%, besonders bevorzugt weniger als 10 Gew.-%, und insbesondere weniger als 8 Gew.-%, jeweils bezogen auf das Gewicht der Komponente a), an Uretonimin. Unter Verwendung einer solchen Isocyanatkomponente a) hergestellte Schaumstoffe weisen einen besonders niedrigen Schrumpf, eine besonders gute Dimensionsstabilität und eine besonders helle Farbe auf.

Die Bestimmung des Gehalts an Uretonimin erfolgt dabei anhand der C=O Schwingung bei 1740cm-1 durch 1,3-Di-p-tolyl-2-p-tolylimino-1,3-diazetidin-4-on Eichung und wird anhand des Molgewichtes auf das Uretonimin des 4,4'-MDI nach Formel (1), umgerechnet

$$\text{mg Uretonimin = Extinktion als Fläche} * 13{,}975 \qquad (1).$$

Vorzugsweise weist die Komponente a) einen Gehalt an freien NCO-Endgruppen von 30 bis 33 Gew.-% auf.

Polyphenylenpolymethylenpolyisocyanat weist üblicherweise 12 bis 18 Gew.-% Uretonimin auf. Es wird üblicherweise hergestellt durch sauer katalysierte Umsetzung von Anilin mit Formaldehyd und Umsetzung des erhaltenen Gemisches aus Diphenylmethandiamin MDA und Polymethylenpolyphenylenpolyamin mit Phosgen zum MDI und nahcfolgende Aufarbeitung und gegebenenfalls partielle Abtrennung des 2-Kem-MDI. Dabei wird die Kondensation mit einem solchen Verhältnis von Anilin zu Formaldehyd durchgeführt, dass sich das gewünschte Verhältnis der Isomere der Homologen im MDA einstellt. Nach der Phosgenierung wird das durch Destillation von flüchtigen Begleitstoffen sowie Nebenprodukten, zumeist Chlor enthaltenden Verbindungen, befreit. Zur Einstellung der Reaktivität kann eine thermische Nachbehandlung erfolgen. Wird diese thermische Belastung durch Destillation und Behandlung auf ein Mindestmaß begrenzt, resultiert ein MDI mit einem Uretonimingehalt bis weniger als 12 Gew.-%, bevorzugt kleiner 10 Gew.-% und besonders bevorzugt kleiner 8 Gew.-%.

Auch bei dem Destillationsschritt zur Einstellung der Komponente a1) muss die thermische Belastung durch Destillation und Behandlung auf ein Mindestmaß begrenzt werden, um die Gehalte an Uretonimin bis weniger als 12 Gew.-%, bevorzugt kleiner 10 Gew.-% und besonders bevorzugt kleiner 8 Gew.-% zu erreichen.

In einer weiteren vorteilhaften Ausführungsform der Erfindung enthalten die Verbindungen mit mindestens zwei mit Isocyanaten reaktiven Wasserstoffatomen mindestens einen Polyetheralkohol b1), der durch Anlagerung von Alkylenoxiden an ein aromatisches Amin hergestellt wurde. Die Hydroxylzahl dieses Polyetheralkohols liegt insbesondere im Bereich von 300 - 500 mg KOH/g. Vorzugsweise wird als aromatisches Amin Toluylendiamin eingesetzt, das besonders bevorzugt zu mindestens 95 Gew.-%, bezogen auf das Gewicht des Toluylendiamins, ortho-isomere enthält.

Weiterhin enthalten die Verbindungen mit mindestens zwei mit Isocyanaten reaktiven Wasserstoffatomen vorzugsweise mindestens einen Polyetheralkohol b2), der durch Anlagerung von Alkylenoxiden an einen Zucker, beispielswesie Sorbit und/oder Saccharose, besonders bevorzugt Saccharose, hergestellt wurde. Die Hydoxylzahl dieses Polyetheralkohols liegt insbesondere im Bereich von 350 - 550 mg KOH/g.

In einer weiteren, besonders bevorzugten Ausführungsform der Erfindung enthalten die Verbindungen mit mindestens zwei mit Isocyanaten reaktiven Wasserstoffatomen mindestens einen Polyetheralkohol b1) und mindestens einen Polyetheralkohol b2). Das Gewichtsverhältnis der Polyole b1) zu b2) liegt vorzugsweise im Bereich von 1:1 bis 1:6.

Zur Herstellung der Polyurethan-Hartschaumstoffe werden die Polyisocyanate a) und die Verbindungen mit mindestens zwei mit Isocyanatgruppen reaktiven Wasserstoffatomen b) und in solchen Mengen zur Umsetzung gebracht, dass der Isocyanatindex in einem Bereich zwischen 100 und 220, vorzugsweise zwischen 110 und 195, liegt.

Die Polyurethan-Hartschaumstoffe können diskontinuierlich oder kontinuierlich mit Hilfe bekannter Mischvorrichtungen hergestellt werden.

Üblicherweise werden die erfindungsgemäßen Polyurethan-Hartschaumstoffe nach dem Zweikomponenten-Verfahren hergestellt. Bei diesem Verfahren werden die Verbindungen mit mindestens zwei gegenüber Isocyanatgruppen reaktiven Wasserstoffatomen b), mit den Flammschutzmitteln, den Treibmitteln, den Katalysatoren sowie den weiteren Hilfs- und/oder Zusatzstoffen zu der sogenannten Polyolkomponente vermischt und diese mit den Polyisocyanaten oder Mischungen aus den Polyisocyanaten und gegebenenfalls Flammschutzmitteln und Treibmitteln, auch als Isocyanatkomponente bezeichnet, zur Umsetzung gebracht.

Die Ausgangskomponenten werden zumeist bei einer Temperatur von 15 bis 35 °C, vorzugsweise von 20 bis 30 °C gemischt. Das Reaktionsgemisch kann mit Hoch- oder Niederdruckdosiermaschinen in geschlossene Stützwerkzeuge gegossen werden. Nach dieser Technologie werden z. B. diskontinuierlich Sandwichelemente gefertigt.

Überraschenderweise weisen die erfindungsgemäßen Polyurethan-Hartschaumstoffe eine sehr gute Entformbarkeit auf. Die Schaumstoffe sind dimensionsstabil und lassen sich sehr gut applizieren.

Zu den übrigen für die Herstellung der erfindungsgemäßen Polyurethan-Hartschaumstoffe eingesetzten Ausgangsverbindungen ist im einzelnen folgendes zu sagen:

Als Verbindungen mit mindestens zwei gegenüber Isocyanat reaktiven Wasserstoffatomen b), die neben den Polyetheralkoholen b1) und b2) für das erfindungsgemäße Verfahren verwendet werden können, kommen insbesondere Polyetheralkohole und/oder Polyesteralkohole mit OH-Zahlen im Bereich von 100 bis 1200 mgKOH/g zum Einsatz.

Die eingesetzten Polyesteralkohole werden zumeist durch Kondensation von mehrfunktionellen Alkoholen, vorzugsweise Diolen, mit 2 bis 12 Kohlenstoffatomen, vorzugsweise 2 bis 6 Kohlenstoffatomen, mit mehrfunktionellen Carbonsäuren mit 2 bis 12 Kohlenstoffatomen, beispielsweise Bernsteinsäure, Glutarsäure, Adipinsäure, Korksäure, Azelainsäure, Sebacinsäure, Decandicarbonsäure, Maleinsäure, Fumarsäure und vorzugsweise Phthalsäure, Isophthalsäure, Terephthalsäure und die isomeren Naphthalindicarbonsäuren, hergestellt.

Die verwendeten Polyetheralkohole haben zumeist eine Funktionalität zwischen 2 und 8, insbesondere 3 bis 8.

Insbesondere kommen Polyetheralkohole, die nach bekannten Verfahren, beispielsweise durch anionische Polymerisation von Alkylenoxiden in Gegenwart von Katalysatoren, vorzugsweise Alkalihydroxiden und/oder Aminen, hergestellt werden, zum Einsatz.

Als Alkylenoxide werden zumeist Ethylenoxid und/oder Propylenoxid, vorzugsweise reines 1,2-Propylenoxid eingesetzt.

Als Startmoleküle kommen insbesondere Verbindungen mit mindestens 3, vorzugsweise 4 bis 8 Hydroxylgruppen oder mit mindestens zwei primären Aminogruppen im Molekül zum Einsatz.

Als Startmoleküle mit mindestens 3, vorzugsweise 4 bis 8 Hydroxylgruppen im Molekül werden vorzugsweise Trimethylolpropan, Glycerin, Pentaerythrit, Zuckerverbindungen, wie beispielsweise Glucose, Sorbit, Mannit und Saccharose, mehrwertige Phenole, Resole, wie z.B. oligomere Kondensationsprodukte aus Phenol und Formaldehyd und Mannich-Kondensate aus Phenolen, Formaldehyd und Dialkanolaminen sowie Melamin eingesetzt.

Als Startmoleküle mit mindestens zwei primären Aminogruppen im Molekül werden vorzugsweise aromatische Di- und/oder Polyamine, beispielsweise Phenylendiamine, 2,3-, 2,4-, 3,4- und 2,6-Toluylendiamin und 4,4'-, 2,4'- und 2,2'-Diaminodiphenylmethan sowie aliphatische Di- und Polyamine, wie Ethylendiamin, eingesetzt.

Die Polyetheralkohole besitzen eine Funktionalität von vorzugsweise 3 bis 8 und Hydroxylzahlen von vorzugsweise 100 mgKOH/g bis 1200 mgKOH/g und insbesondere 240 mgKOH/g bis 570 mgKOH/g. Es können zusätzlich auch Polyole, insbesondere Polyetheralkohole mit einer Hydroxylzahl von 100 - 250 mgKOH/g und einer Funktionalität von 2,5 bis 4 eingesetzt werden. Dadurch können die Eigenschaften der Schaumstoffe eingestellt werden.

Zu den Verbindungen mit mindestens zwei gegenüber Isocyanat reaktiven Wasserstoffatomen (A) gehören auch die gegebenenfalls mitverwendeten Kettenverlängerer und Vernetzer. Zur Modifizierung der mechanischen Eigenschaften kann sich der Zusatz von difunktionellen Kettenverlängerungsmitteln, tri- und höherfunktionellen Vernetzungsmitteln oder gegebenenfalls auch Gemischen davon als vorteilhaft erweisen. Als Kettenverlängerungs- und/oder Vernetzungsmittel verwendet werden vorzugsweise Alkanolamine und insbesondere Diole und/oder Triole mit Molekulargewichten kleiner als 400, vorzugsweise 60 bis 300.

Kettenverlängerungsmittel, Vernetzungsmittel oder Mischungen davon werden zweckmäßigerweise in einer Menge von 1 bis 20 Gew.-%, vorzugsweise 2 bis 10 Gew.-%, bezogen auf die Komponente b), eingesetzt.

Weitere Angaben zu den verwendeten Polyetheralkoholen und Polyesteralkoholen sowie ihrer Herstellung finden sich beispielsweise im Kunststoffhandbuch, Band 7 "Polyurethane", herausgegeben von Günter Oertel, Carl-Hanser-Verlag München, 3. Auflage, 1993.

Als Treibmittel c) kann Wasser verwendet werden, das mit Isocyanatgruppen unter Abspaltung von Kohlendioxid reagiert. In Kombination mit oder an Stelle von Wasser können auch sogenannte physikalische Treibmittel eingesetzt werden. Dabei handelt es sich um gegenüber den Einsatzkomponenten inerte Verbindungen, die zumeist bei Raumtemperatur flüssig sind und bei den Bedingungen der Urethanreaktion verdampfen. Vorzugsweise liegt der Siedepunkt dieser Verbindungen unter 50 °C. Zu den physikalischen Treibmitteln zählen auch Verbindungen, die bei Raumtemperatur gasförmig sind und unter Druck in die Einsatzkomponenten eingebracht bzw. in ihnen gelöst werden, beispielsweise Kohlendioxid, niedrigsiedende Alkane und Fluoralkane oder Fluoralkene.

Diese Verbindungen werden zumeist ausgewählt aus der Gruppe, enthaltend Alkane und/oder Cycloalkane mit mindestens 4 Kohlenstoffatomen, Dialkylether, Ester, Ketone, Acetale, Fluoralkane oder Fluoralkene mit 1 bis 8 Kohlenstoffatomen, und Tetraalkylsilane mit 1 bis 3 Kohlenstoffatomen in der Alkylkette, insbesondere Tetramethylsilan.

Als Beispiele seien genannt Propan, n-Butan, iso- und Cyclobutan , n-, iso- und Cyclopentan, Cyclohexan, Dimethylether, Methylethylether, Methylbutylether, Ameisensäuremethylester, Aceton, sowie Fluoralkane, die in der Troposphäre abgebaut werden können und deshalb für die Ozonschicht unschädlich sind, wie Trifluormethan, Difluormethan, 1,1,1,3,3-Pentafluorbutan, 1,1,1,3,3-Pentafluorpropan, 1,1,1,2- Tetrafluorethan, Difluorethan und Heptafluorpropan. Besonders bevorzugt eingesetzt werden Cyclopentan und/oder n-Pentan. Die genannten physikalischen Treibmittel können allein oder in beliebigen Kombinationen untereinander eingesetzt werden.

Als Katalysatoren werden insbesondere Verbindungen eingesetzt, welche die Reaktion der Isocyanatgruppen mit den mit Isocyanatgruppen reaktiven Gruppen stark beschleunigen. Solche Katalysatoren sind stark basische Amine, wie z. B. tertäre aliphatische Amine, Imidazole, Amidine, sowie Alkanolamine.

Zur Herstellung der Polyurethan-Hartschaumstoffe werden die Verbindungen mit mindestens zwei mit Isocyanaten reaktiven Wasserstoffatomen b) und die Polyisocyanate a) und in solchen Mengen zur Umsetzung gebracht, dass der Isocyanatindex in einem Bereich zwischen 100 und 220, vorzugsweise zwischen 110 und 195, liegt.

Falls in den Hartschaumstoff Isocyanuratgruppen eingebaut werden sollen, werden spezielle Katalysatoren benötigt. Als Isocyanurat-Katalysatoren werden üblicherweise Metallcarboxylate, insbesondere Kaliumacetat und dessen Lösungen, eingesetzt. Bei der Herstellung derartiger Schäume, auch als Polyurethan-Polyisocyanurat-Schäume bezeichnet, wird die Umsetzung der Komponenten (A) und (B) üblicherweise bei einem Index von 160 bis 450 durchgeführt

Üblicherweise werden die erfindungsgemäßen PUR-Hartschaumstoffe nach dem Zweikomponenten-Verfahren hergestellt. Bei diesem Verfahren werden die Verbindungen mit mindestens zwei gegenüber Isocyanatgruppen reaktiven Wasserstoffatomen (A), mit den Flammschutzmitteln, den Treibmitteln, den Katalysatoren sowie den weiteren Hilfs- und/oder Zusatzstoffen zu der sogenannten Polyolkomponente vermischt und diese mit den Polyisocyanaten oder Mischungen aus den Polyisocyanaten und gegebenenfalls Flammschutzmitteln und Treibmitteln, auch als Isocyanatkomponente bezeichnet, zur Umsetzung gebracht.

Die Ausgangskomponenten werden zumeist bei einer Temperatur von 15 bis 35 °C, vorzugsweise von 20 bis 30 °C gemischt. Das Reaktionsgemisch kann mit Hoch- oder Niederdruckdosiermaschinen in geschlossene Stützwerkzeuge gegossen werden. Nach dieser Technologie werden z. B. diskontinuierlich Sandwichelemente gefertigt.

Die Polyurethan-Hartschaumstoffe können diskontinuierlich oder kontinuierlich mit Hilfe bekannter Mischvorrichtungen hergestellt werden.

Überraschenderweise weisen die nach dem erfindungsgemäßen Verfahren hergestellten Schaumstoffe eine gute Entformbarkeit auf. Die Schaumstoffe sind dimensionsstabil und lassen sich sehr gut applizieren.

Die nach dem erfindungsgemäßen Verfahren hergestellten Polyurethan-Hartschaumstoffe weisen bei der ausschließlichen Verwendung von Wasser als Treibmittel eine Dichte von kleiner 38 g/l auf. Bei der Mitverwendung von physikalischen Treibmitteln liegt die Dichte der Polyurethan-Hartschaumstoffe vorzugsweise im Bereich zwischen 28 und 33 g/l.

Auch bei diesen niedrigen Dichten tritt bei den erfindungsgemäßen Polyurethan-Hartschaumstoffen überraschenderweise keinerlei Schrumpf auf.

Die Erfindung soll an den nachfolgenden Beispielen näher erläutert werden.

1.1 Herstellung der Polyolkomponente 1:

Aus 520g eines Polyetherpolyols auf Basis Saccharose, Glycerin und Propylenoxid mit einem mittleren Molekulargewicht von 640 g/mol, 250g eines Polyetheralkohols auf Basis vicinalem TDA, Ethylenoxid und Propylenoxid mit einem mittleren Molekulargewicht von 575 g/mol, 160g eines Polyetheralkohols auf Basis viccinalem TDA, Ethylenoxid und Propylenoxid mit einem mittleren Molekulargewicht von 1400 g/mol, 20g eines Schaumstabilisators, 16,5g Polycat 8, 6,5g Polycat 5, 3,5g Polycat 41, 24g Wasser und 14g Cyclopentan wurde durch Vermischen eine Polyolkomponente hergestellt.

1.2 Herstellung der Polyolkomponente 2:

Aus 520g eines Polyetheralkohols auf Basis Saccharose, Glycerin und Propylenoxid mit einem mittleren Molekulargewicht von 640 g/mol, 250g eines Polyetheralkohols auf Basis vicinalem TDA, Ethylenoxid und Propylenoxid mit einem mittleren Molekulargewicht von 575 g/mol, 160g eines Polyetheralkohols auf Basis vicinalem TDA, Ethylenoxid und Propylenoxid mit einem mittleren Molekulargewicht von 1400 g/mol, 20g eines Schaumstabilisators, 16,5g Polycat 8, 6,5g Polycat 5, 3,5g Polycat 41, 24g Wasser und 17g Cyclopentan wurde durch Vermischen eine Polyolkomponente hergestellt.

1.3 Herstellung des PU-Hartschaums (nicht erfindungsgemäß)

Polyphenylenpolymethylenpolyisocyanat (Handelsname: Lupranat® M20S) mit einem Monomer-MDI-Gehalt von 39 %, 29 Gew.-% Polyphenylenpolymethylenpolyisocyanat mit drei aromatischen Ringen, 12 Gew.-% Polyphenylenpolymethylenpolyisocyanat mit vier aromatischen Ringen, 20 Gew.-% Polyphenylenpolymethylenpolyisocyanat mit fünf oder mehr aromatischen Ringen, einem NCO-Gehalt von 31,2 Gew.-%, einer Viskosität von 252 mPa·s bei 25 °C und einem Uretonimin-Gehalt von 14% wurde mit der Polyolkomponente 1 im Verhältnis Polyol : Isocyanat = 100 : 121 mit einer Hochdruckdosiermaschine vermischt. Diese Mischung wurde in eine Lanzen-Form (2000x200x50mm) oder eine Kasten-Form (700x400x90mm) injiziert.

Man erhielt ein PU - Hartschaum, welche sich wie folgt charakterisieren lässt:

Abbindezeit : 41sec

Rohdichte : 35g/l

Entformdicke nach 4min bei 15% Überfüllung: 93 mm

Fließfaktor : 1,38

Wärmeleitfähigkeit bei 23°C : 19,0 mW/mK

Dimensionsstabilität nach DIN EN 1604/13165 (Stufe 12): erfüllt

bei 70°C, 90% rel.F.:Länge: 0% , Breite : 0,85% , Höhe : 0,35%

bei -20°C : Länge : -0,05% , Breite : 0% Höhe : -0,1 %

1.4 Herstellung des PU-Hartschaums (erfindungsgemäß)

Polyphenylenpolymethylenpolyisocyanat mit einem Monomer-MDI-Gehalt von 33 %, 25 Gew.-% Polyphenylenpolymethylenpolyisocyanat mit drei aromatischen Ringen, 10 Gew.-% Polyphenylenpolymethylenpolyisocyanat mit vier aromatischen Ringen, 32 Gew.-% Polyphenylenpolymethylenpolyisocyanat mit fünf oder mehr aromatischen Ringen, einem NCO-Gehalt von 31,5 Gew.-%, einer Viskosität von 355 mPa.s bei 25 °C und einem Uretonimin-Gehalt von 9% wurde mit der Polyolkomponente 1 im Verhältnis Polyol : Isocyanat = 100 : 123 mit einer Hochdruckdosiermaschine vermischt. Diese Mischung wurde in eine Lanzen-Form (2000x200x50mm) oder eine Kasten-Form (700x400x90mm) injiziert.

Man erhielt ein PU - Hartschaum, welche sich wie folgt charakterisieren lässt:

Abbindezeit : 40sec

Rohdichte : 29 g/l

Entformdicke nach 4min bei 15% Überfüllung: 92,2 mm

Fließfaktor : 1,40

Wärmeleitfähigkeit bei 23°C : 18,7 mW/mK

Dimensionsstabilität nach DIN EN 1604/13165 (Stufe 12): erfüllt

bei 70°C, 90% rel.F.: Länge : 0,2% , Breite : 0,9% , Höhe : 1,1%

bei -20°C : Länge : 0% , Breite : 0,1 % , Höhe : 0.1 %

**Patentansprüche**

**1.** Polyurethan-Hartschaumstoffe, herstellbar durch Umsetzung von

a) Polyisocyanaten mit

b) Verbindungen mit mindestens zwei mit Isocyanaten reaktiven Wasserstoffatomen in Gegenwart von
c) Treibmitteln,

**dadurch gekennzeichnet, dass** als Polyisocyanat a) eine Mischung aus Diphenylmethandiisocyanat und Polyphenylenpolymethylenpolyisocyanaten, enthaltend,

a1) 30 - 35 Gew.-% Diphenylmethandiisocyanat
a2) 21 - 28 Gew.-% Polyphenylenpolymethylenpolyisocyanat mit drei aromatischen Ringen,
a3) 8 - 13 Gew.-% Polyphenylenpolymethylenpolyisocyanat mit vier aromatischen Ringen,
a4) 24 - 41 Gew.-% Polyphenylenpolymethylenpolyisocyanat mit fünf oder mehr aromatischen Ringen,

wobei sich die Gew.-% der Komponenten a1) bis a4) auf das Gesamtgewicht der Komponenten a1) bis a4) beziehen und sich zu 100 Gew.-% ergänzen, verwendet wird und wobei die Komponente a) weniger als 12 Gew.-%, bezogen auf das Gewicht der Komponente a), an Uretonimin enthält.

2. Polyurethan-Hartschaumstoffe nach Anspruch 1, **dadurch gekennzeichnet, dass** die Komponente a) weniger als 10 Gew.-%, bezogen auf das Gewicht der Komponente a), an Uretonimin enthält.

3. Polyurethan-Hartschaumstoffe nach Anspruch 1, **dadurch gekennzeichnet, dass** die Komponente a) weniger als 8 Gew.-%, bezogen auf das Gewicht der Komponente a), an Uretonimin enthält.

4. Polyurethan-Hartschaumstoffe nach Anspruch 1, **dadurch gekennzeichnet, dass** die Komponente a) einen Gehalt an freien NCO-Endgruppen von 30 bis 33 Gew.-% aufweist.

5. Polyurethan-Hartschaumstoffe nach Anspruch 1, **dadurch gekennzeichnet, dass** sie Isocyanuratgruppen enthalten.

6. Polyurethan-Hartschaumstoffe nach Anspruch 1, **dadurch gekennzeichnet, dass** die Komponente b) mindestens einen Polyetheralkohol b1) enthält, der durch Anlagerung von Alkylenoxiden an ein aromatisches Amin hergestellt wurde.

7. Polyurethan-Hartschaumstoffe nach Anspruch 6, **dadurch gekennzeichnet, dass** der Polyetheralkohol b1) durch Anlagerung von Alkylenoxiden an Toluylendiamin hergestellt wurde.

8. Polyurethan-Hartschaumstoffe nach Anspruch 7, **dadurch gekennzeichnet, dass** das Toluylendiamin zu mindestens 95 Gew.-%, bezogen auf das Gewicht des Toluylendiamins, ortho-Isomere enthält.

9. Polyurethan-Hartschaumstoffe nach Anspruch 1, **dadurch gekennzeichnet, dass** die Komponente b) mindestens einen Polyetheralkohol b2) enthält, der durch Anlagerung von Alkylenoxiden an einen Zucker hergestellt wurde.

10. Polyurethan-Hartschaumstoffe nach Anspruch 9, **dadurch gekennzeichnet, dass** der Polyetheralkohol b2) durch Anlagerung von Alkylenoxiden an Saccharose hergestellt wurde.

11. Polyurethan-Hartschaumstoffe nach Anspruch 1, **dadurch gekennzeichnet, dass** die Komponente b) mindestens einen Polyetheralkohol b1) und einen Polyetheralkohol b2) enthält

12. Verfahren zur Herstellung von Polyurethan-Hartschaumstoffen durch Umsetzung von

a) Polyisocyanaten mit
b) Verbindungen mit mindestens zwei mit Isocyanaten reaktiven Wasserstoffatomen in Gegenwart von
c) Treibmitteln,

**dadurch gekennzeichnet, dass** als Polyisocyanat a) eine Mischung aus Diphenylmethandiisocyanat und Polyphenylenpolymethylenpolyisocyanaten, enthaltend,

a1) 30 - 35 Gew.-% Diphenylmethandiisocyanat
a2) 21 - 28 Gew.-% Polyphenylenpolymethylenpolyisocyanat mit drei aromatischen Ringen,
a3) 8 - 13 Gew.-% Polyphenylenpolymethylenpolyisocyanat mit vier aromatischen Ringen,

a4) 24 - 41 Gew.-% Polyphenylenpolymethylenpolyisocyanat mit fünf oder mehr aromatischen Ringen,

wobei sich die Gew.% der Komponenten a1) bis a4) auf das Gesamtgewicht der Komponenten a1) bis a4) beziehen und sich zu 100 Gew.-% ergänzen, verwendet wird und wobei die Komponente a) weniger als 12 Gew.-%, bezogen auf das Gewicht der Komponente a), an Uretonimin enthält.

## Claims

1. A rigid polyurethane foam which can be produced by reacting

    a) polyisocyanates with
    b) compounds having at least two hydrogen atoms which are reactive toward isocyanate groups in the presence of
    c) blowing agents,

    wherein the polyisocyanate a) used is a mixture of diphenylmethane diisocyanate and polyphenylenepolymethylene polyisocyanates comprising

    a1) 30 - 35% by weight of diphenylmethane diisocyanate,
    a2) 21 - 28% by weight of polyphenylenepolymethylene polyisocyanate having three aromatic rings,
    a3) 8 - 13% by weight of polyphenylenepolymethylene polyisocyanate having four aromatic rings,
    a4) 24 - 41% by weight of polyphenylenepolymethylene polyisocyanate having five or more aromatic rings,

    where the percentages by weight of the components a1) to a4) are based on the total weight of the components a1) to a4) and add up to 100% by weight and the component a) comprises less than 12% by weight, based on the weight of the component a), of uretonimine.

2. The rigid polyurethane foam according to claim 1, wherein the component a) comprises less than 10% by weight, based on the weight of the component a), of uretonimine.

3. The rigid polyurethane foam according to claim 1, wherein the component a) comprises less than 8% by weight, based on the weight of the component a), of uretonimine.

4. The rigid polyurethane foam according to claim 1, wherein the component a) has a content of free NCO end groups of from 30 to 33% by weight.

5. The rigid polyurethane foam according to claim 1 which comprises isocyanurate groups.

6. The rigid polyurethane foam according to claim 1, wherein the component b) comprises at least one polyether alcohol b1) which has been prepared by addition of alkylene oxides onto an aromatic amine.

7. The rigid polyurethane foam according to claim 6, wherein the polyether alcohol b1) has been prepared by addition of alkylene oxides onto toluenediamine.

8. The rigid polyurethane foam according to claim 7, wherein the toluenediamine comprises at least 95% by weight, based on the weight of the toluenediamine, of ortho isomers.

9. The rigid polyurethane foam according to claim 1, wherein the component b) comprises at least one polyether alcohol b2) which has been prepared by addition of alkylene oxides onto a sugar.

10. The rigid polyurethane foam according to claim 9, wherein the polyether alcohol b2) has been prepared by addition of alkylene oxides onto sucrose.

11. The rigid polyurethane foam according to claim 1, wherein the component b) comprises at least one polyether alcohol b1) and a polyether alcohol b2).

12. A process for producing rigid polyurethane foams by reacting

a) polyisocyanates with

b) compounds having at least two hydrogen atoms which are reactive toward isocyanate groups in the presence of

c) blowing agents,

wherein the polyisocyanate a) used is a mixture of diphenylmethane diisocyanate and polyphenylenepolymethylene polyisocyanates comprising

a1) 30 - 35% by weight of diphenylmethane diisocyanate,

a2) 21 - 28% by weight of polyphenylenepolymethylene polyisocyanate having three aromatic rings,

a3) 8 - 13% by weight of polyphenylenepolymethylene polyisocyanate having four aromatic rings,

a4) 24 - 41% by weight of polyphenylenepolymethylene polyisocyanate having five or more aromatic rings,

where the percentages by weight of the components a1) to a4) are based on the total weight of the components a1) to a4) and add up to 100% by weight and the component a) comprises less than 12% by weight, based on the weight of the component a), of uretonimine.

## Revendications

1. Mousses dures de polyuréthanne, pouvant être fabriquées par réaction de

a) des polyisocyanates avec

b) des composés contenant au moins deux atomes d'hydrogène réactifs avec les isocyanates, en présence de

c) des agents gonflants,

**caractérisées en ce qu'**un mélange de diisocyanate de diphénylméthane et de polyisocyanates de polyphénylène-polyméthylène contenant

a1) 30 à 35 % en poids de diisocyanate de diphénylméthane

a2) 21 à 28 % en poids de polyisocyanate de polyphénylène-polyméthylène contenant trois cycles aromatiques,

a3) 8 à 13 % en poids de polyisocyanate de polyphénylène-polyméthylène contenant quatre cycles aromatiques,

a4) 24 à 41 % en poids de polyisocyanate de polyphénylène-polyméthylène contenant cinq cycles aromatiques ou plus,

est utilisé en tant que polyisocyanate a), les % en poids des composants a1) à a4) se fondant sur le poids total des composants a1) à a4) et totalisant 100 % en poids et le composant a) contenant moins de 12 % en poids, par rapport au poids du composant a), d'urétonimine.

2. Mousses dures de polyuréthanne selon la revendication 1, **caractérisées en ce que** le composant a) contient moins de 10 % en poids, par rapport au poids du composant a), d'urétonimine.

3. Mousses dures de polyuréthanne selon la revendication 1, **caractérisées en ce que** le composant a) contient moins de 8 % en poids, par rapport au poids du composant a), d'urétonimine.

4. Mousses dures de polyuréthanne selon la revendication 1, **caractérisées en ce que** le composant a) a une teneur en groupes terminaux NCO libres de 30 à 33 % en poids.

5. Mousses dures de polyuréthanne selon la revendication 1, **caractérisées en ce qu'**elles contiennent des groupes isocyanurate.

6. Mousses dures de polyuréthanne selon la revendication 1, **caractérisées en ce que** le composant b) contient au moins un polyétheralcool b1) qui a été fabriqué par addition d'oxydes d'alkylène sur une amine aromatique.

7. Mousses dures de polyuréthanne selon la revendication 6, **caractérisées en ce que** le polyétheralcool b1) a été fabriqué par addition d'oxydes d'alkylène sur une toluylènediamine.

8. Mousses dures de polyuréthanne selon la revendication 7, **caractérisées en ce que** la toluylènediamine contient au moins 95 % en poids, par rapport au poids de la toluylènediamine, d'isomères ortho.

9. Mousses dures de polyuréthanne selon la revendication 1, **caractérisées en ce que** le composant b) contient au moins un polyétheralcool b2) qui a été fabriqué par addition d'oxydes d'alkylène sur un sucre.

10. Mousses dures de polyuréthanne selon la revendication 9, **caractérisées en ce que** le polyétheralcool b2) a été fabriqué par addition d'oxydes d'alkylène sur du saccharose.

11. Mousses dures de polyuréthanne selon la revendication 1, **caractérisées en ce que** le composant b) contient au moins un polyétheralcool b1) et un polyétheralcool b2).

12. Procédé de fabrication de mousses dures de polyuréthanne par réaction de

a) des polyisocyanates avec
b) des composés contenant au moins deux atomes d'hydrogène réactifs avec les isocyanates, en présence de
c) des agents gonflants,

**caractérisé en ce qu'**un mélange de diisocyanate de diphénylméthane et de polyisocyanates de polyphénylène-polyméthylène contenant

a1) 30 à 35 % en poids de diisocyanate de diphénylméthane
a2) 21 à 28 % en poids de polyisocyanate de polyphénylène-polyméthylène contenant trois cycles aromatiques,
a3) 8 à 13 % en poids de polyisocyanate de polyphénylène-polyméthylène contenant quatre cycles aromatiques,
a4) 24 à 41 % en poids de polyisocyanate de polyphénylène-polyméthylène contenant cinq cycles aromatiques ou plus,

est utilisé en tant que polyisocyanate a), les % en poids des composants a1) à a4) se fondant sur le poids total des composants a1) à a4) et totalisant 100 % en poids et le composant a) contenant moins de 12 % en poids, par rapport au poids du composant a), d'urétonimine.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0294110 A

**In der Beschreibung aufgeführte Nicht-Patentliteratur**

- Polyurethane. Kunststoffhandbuch. Carl-Hanser-Verlag, 1993, vol. 7